(19)

Europäisches
Patentamt
European
Patent Office
Office européen
des brevets

(11) **EP 2 596 776 A1**

(12) **EUROPEAN PATENT APPLICATION**

(43) Date of publication:
   **29.05.2013 Bulletin 2013/22**

(51) Int Cl.:
   *A61F 13/00* (2006.01)   *A61L 15/36* (2006.01)

(21) Application number: **11190803.4**

(22) Date of filing: **25.11.2011**

(84) Designated Contracting States:
   **AL AT BE BG CH CY CZ DE DK EE ES FI FR GB GR HR HU IE IS IT LI LT LU LV MC MK MT NL NO PL PT RO RS SE SI SK SM TR**
   Designated Extension States:
   **BA ME**

(71) Applicant: **Micuri ApS**
   **3400 Hillerød (DK)**

(72) Inventors:
   • **NIELSEN, Brian**
     **3330 Gørløse (DK)**
   • **Nielsen, Erik**
     **3000 Frederiksvaerk (DK)**

(74) Representative: **Østergaard, Steen**
   **Guardian IP Consulting I/S**
   **Diplomvej, Building 381**
   **2800 Kgs. Lyngby (DK)**

(54) **A packaged probiotic composition and uses thereof**

(57)    The present invention relates to a packaged probiotic composition which is useful for treating or preventing bacterial colonisation in wounds and tissue. A characteristic feature of the invention is that the probiotic composition contains a probiotic microorganism which is in direct contact with water during storage of the probiotic composition. The invention furthermore pertains to methods of using and producing the probiotic composition.

# Fig. 3b

**Description**

**FIELD OF THE INVENTION**

The present invention relates to a packaged probiotic composition which is useful for treating or preventing bacterial colonisation in wounds and tissue. A characteristic feature of the invention is that the probiotic composition contains a probiotic microorganism which is in direct contact with water during storage of the probiotic composition. The invention furthermore pertains to methods of using and producing the probiotic composition.

**BACKGROUND OF THE I NVENTI ON**

It has been known for decades that probiotic bacteria improve the health of the digestive system, also if inflammation or wounds occur in the bowel (treating Cullitis Ulcerosa or Morcus Chrom).
Some prior art documents furthermore describe probiotic compositions for topical applications.
WO 2008/074,331 discloses the use of lyophilized lactic acid bacteria in wound dressings. Such wound dressings may also contain absorbent materials for absorbing exudates from the wound. WO 2008/074,331 also discloses that such wound dressings may contain water, but it does not describe that lactic acid bacteria are in contact with water during storage.
WO 00/61,201 discloses a method of inhibiting microbial infection which is often observed in connection with the use of diapers and other sanitary products. More specifically, WO 00/61,201 describes that an aqueous suspension of lactic acid-producing bacteria is applied to the sanitary product, the product is dried and the sanitary product is subsequently used.
However, it does not describe that lactic acid-producing bacteria of the sanitary product are in contact with water during storage of the sanitary product.

**SUMMARY OF THE INVENTION**

The present inventor has realised that the above-mentioned approaches of the prior art suffer from significant draw-backs.
The use of dried probiotic microorganisms reported in the prior art results in a substantial loss of viability of the microorganisms. Yet a disadvantage of this approach is that it takes time for the viable microorganisms to recover when resuspended after having been stored in dry form for a long time. This lag time delays the beneficial, infection-reducing effect provided by the probiotic microorganisms and reduces the medical value of the product.
The present inventor has discovered a new type of probiotic composition in which the probiotic microorganisms are present in an aqueous medium during storage and which has a shelf life of more than 3 month at 23 degrees C.
Thus, an aspect of the invention relates to a packaged probiotic composition, the probiotic composition comprising

- a water-containing composition containing a viable first probiotic microorganism and

- a support agent.

In some preferred embodiments of the invention, the support agent is in direct contact with the water-containing composition in the probiotic composition during storage. The support agent may for example have absorbed the water-containing composition or the probiotic composition may comprise a hydrogel which contains the support agent and the water-containing composition.
The inventor has discovered that for such embodiments, surprisingly, the stability of the support agent in the water-containing composition is important to obtain a long shelf-life of the probiotic composition. Furthermore, it may be desirable that the support agent does not degrade when applied to a wound. In such embodiments it is therefore preferred that the support agent is non-degradable by the water-containing composition.
Yet an aspect of the present invention pertains to a method of producing a packaged probiotic composition, the method comprising the steps of:

a) providing a water-containing composition containing a viable first probiotic microorganism,

b) providing a support agent,

c) optionally, contacting the support agent with the water-containing composition, and

d) packaging the combination of the support agent and the water-containing composition in a suitable container.

As stated above the support agent may in some embodiments of the invention be non-degradable by the water-containing composition.

Another aspect of the present invention pertains to a method of treating a human or animal subject having a colonized wound or tissue, or being at risk of having a colonized wound or tissue, the method comprising the steps of:

1) providing a packaged probiotic composition as described herein,

2) opening the container in which the probiotic composition has been packaged, and

3) applying the probiotic composition to the colonized wound or tissue.

A further aspect of the invention pertains to a probiotic composition described herein for use in treatment or prevention of colonized wound or tissue.

## BRIEF DESCRIPTION OF THE FIGURES

Figure 1 is a schematic illustration of a cross section of a pad wound dressing-type probiotic composition according to the invention,

Figure 2a is a schematic illustration of a pad dressing-type probiotic composition (1) which contains a reservoir containing the water-containing composition (6) and a separate support agent (8),

Figure 2b is a schematic illustration of the pad dressing-type probiotic composition (1) of Figure 2a, wherein the water-containing composition of the reservoir (6) has been transferred the support agent (2),

Figure 3a is a schematic illustration of a cross section of the components used to prepare an adhesive dressing-type probiotic composition,

Figure 3b is a schematic illustration of an adhesive dressing-type probiotic composition (1),

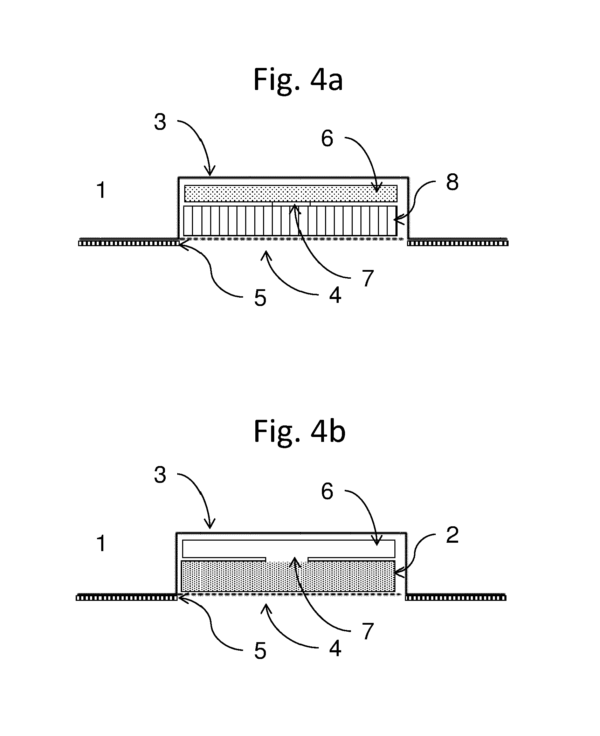

Figure 4a is a schematic illustration of an adhesive dressing-type probiotic composition (1) which contains a reservoir containing the water-containing composition (6) and a separate support agent (8), and

Figure 4b is a schematic illustration of the adhesive dressing-type probiotic composition (1) of Figure 4a, wherein the water-containing composition of the reservoir (6) has been transferred the support agent (9).

## DETAILED DESCRIPTION OF THE INVENTION

As mentioned, an aspect of the invention pertains to a packaged probiotic composition, the probiotic composition comprising

- a water-containing composition containing a viable first probiotic microorganism, and

- a support agent.

The probiotic composition may be a semi-homogeneous composition such as a hydrogel consisting essentially to the water-containing composition and the support agent, or it may be a structured composition containing a number of structural components in addition to the support agent and the water-containing composition. An example of such a structured probiotic composition is an adhesive wound dressing, which, in addition to the support agent and the water-containing composition, may contain a top layer to which the support agent and an adhesive layer are attached.

In the context of the present invention, the terms "consists essentially of" or "consisting essentially of" means that the process or product in question consists of the specified materials or steps and those that do not materially affect the basic and novel characteristic(s) of the invention.

In the context of the present invention, the term "packaged probiotic composition" pertains to a probiotic composition enclosed within a container that serves to protect the probiotic composition from contamination from the environment. Preferably the container forms a sterile barrier seal from the external non-sterile environment. The sterile seal is preferably first broken when the probiotic composition is used, for example as a wound dressing.

The water-containing composition comprises a first probiotic microorganism that is characterised as a viable microorganism, which when administered to a host can confer a health benefit by virtue of its ability to displace, inhibit and/or destroy a pathogenic microorganism. The probiotic microorganism has the capacity to inhibit and/or prevent colonization by a pathogenic microorganism in a wound or a tissue when applied to that wound or tissue.

In the context of the present invention, the phrase "Y and/or X" means "Y" or "X" or "Y and X". Along the same line of logic, the phrase "$n_1$, $n_2$, ..., $n_{i-1}$, and/or $n_1$" means " $n_1$" or "$n_2$" or ... or "$n_{i-1}$" or "$n_i$" or any combination of the components : $n_1$, $n_2$,...$n_{i-1}$, and $n_i$.

The probiotic microorganism in the water-containing composition is viable and is thus at least capable of being metabolically active and preferably also capable of growing. The probiotic microorganism preferably has the capacity of metabolic activity and growth when used as a component of the probiotic composition and when brought in contact with one or more metabolisable substrates or nutrients present in exudate from the wound or tissue.

The water-containing composition is preferably a water-containing liquid and typically contains a significant amount of water. In preferred embodiments of the invention, the water-containing composition contains water in an amount of at least 50% (w/w) relative to the total weight of the water-containing composition.

For example, the water-containing composition may contain water in an amount of at least 60% (w/w), such as at least 70% (w/w), preferably at least 80% (w/w), and even more preferred at least 90% (w/w).

It may be preferred that the water-containing composition contains water in an amount of at least 95% (w/w), such as at least 97% (w/w).

The water-containing composition may for example contain an amount of water in the range of 50% - 99.9% (w/w), such as in the range of 60% - 99.7% (w/w), preferably in the range of 70% - 99.5% (w/w), such as in the range of 80% - 99.3% (w/w), and even more preferably in the range of 90% - 99.1% (w/w) such as in the range of 95% - 99% (w/w).

In preferred embodiments of the invention, the first probiotic microorganism is a bacterium or a fungus. It is particularly preferred that the first probiotic microorganism is a bacterium.

It is preferred that the bacterium is an anaerobic bacterium, for example a lactic acid producing bacterium such as e.g. a *Lactobacillus* species or a *Bifodobacterium species.*

In some preferred embodiments of the invention, the first probiotic microorganism is capable of producing one or more bacteriocin(s) (e.g subspecies of *Lactobacillus lactic* and *Lactobacillus brevis*). A bacteriocin-producing microorganism is advantageous in the context of the present invention, since bacteriocins are known as narrow spectrum antibiotics that act as proteinaceous toxins and inhibit the growth of other bacterial strains.

In some embodiments of the present invention, the probiotic microorganism is a lactic acid bacterium. The lactic acid bacterium may for example be a bacterium belonging to genus selected from the group consisting of a *Carnobacterium, an Enterococcus, a Lactobacillus, a Lactococcus, a Leuconostoc, an Oenococcus, a Pediococcus, a Streptococcus, a Tetragenococcus, a Vaggococcus, a Weissella* a and Bifodobacterium. [Er disse alle lactic acid bacteria?]

In some embodiments of the present invention, the first probiotic microorganism is a Bacillus strain (e.g. B. coagulans).

In other embodiments of the present invention, the first probiotic microorganism is a yeast, e.g. such as *Saccharomyces boulardii, Saccharomyces cerevisiae* or

*Aspergillus oryzae.*

Other useful lactic acid bacteria and non-lactic acid bacteria may be found in WO 2008/074,331 or WO 00/61,201.

The water-containing composition may contain one or more additional probiotic microorganisms selected from the microorganisms mentioned above. The water-containing composition may for example contain a second probiotic microorganism, which is different to the first probiotic microorganism, and even a third probiotic microorganism which is different from the first and second probiotic microorganism.

Additionally, the water-containing composition may also contain one or more non-probiotic microorganisms (e.g. non-probiotic lactic acid bacteria).

However, it is preferred that neither the water-containing composition nor the probiotic composition as such contains pathogenic microorganisms.

In some preferred embodiments of the invention, the water-containing composition contains a total amount of probiotic microorganisms of at least about $10^3$ cfu (colony forming units) per mL. The probiotic composition may for example contain a total amount of probiotic microorganisms of at least about $10^4$ cfu, preferably at least $10^5$ cfu, such as at least $10^6$ cfu, or at least $10^7$ cfu, and even more preferably at least $10^8$ cfu, of probiotic microorganism per mL, such as at least $10^9$ cfu per mL.

Alternatively, the water-containing composition may contain a total amount of probiotic microorganisms between $10^3$ cfu and $10^{10}$ cfu per mL, preferably between $10^5$ cfu and $10^9$ cfu, and even more preferably between $10^6$ cfu and

$10^8$ cfu, of probiotic microorganisms per mL. As will be understood by the person skilled in the art, the total amount of probiotic microorganisms encompasses all individual cells of the first probiotic microorganism as well as the individual cells of any additional probiotic microorganisms.

The present inventor has discovered that storage of packaged probiotic microorganisms sometimes is associated with undesirable gas production in the packaged probiotic composition. Even a moderate degree of gas production may lead to the formation of gas bubbles in the support agent or elsewhere in the probiotic composition, which may reduce the overall efficiency of the probiotic composition. A large degree of gas production is capable of expanding, and even exploding, the packaging in which the probiotic composition has been packaged, in which case the probiotic composition is rendered useless.

The present inventor has furthermore discovered that the problem associated with undesirable gas development can be solved by limiting to amount of low-molecular carbon-containing nutrients in the water-containing composition.

Thus, in some preferred embodiments of the invention, the total amount of carbon-containing nutrients having a molecular weight of at most 5000 g/mol in the water-containing composition is at most 0.5% (w/w) relative to the weight of the water-containing composition. For example, the total amount of carbon-containing nutrients having a molecular weight of at most 5000 g/mol in the water-containing composition may be at most 0.2% (w/w) relative to the weight of the water-containing composition, preferably at most 0.1% (w/w), and even more preferably at most 0.05% (w/w) relative to the weight of the water-containing composition, such as at most 0.001% (w/w).

In the context of the present invention, the term "carbon-containing nutrients having a molecular weight of at most 5000 g/mol" pertains to organic compounds that act as nutrients and carbon source to the first probiotic microorganism. Such compounds are e.g. alcohols, carbohydrates, peptides, fatty acids and combinations thereof. It should be noted that carbon-containing compounds, which have no nutritional value to the probiotic microorganism(s) are not perceived as carbon-containing nutrients according to the present invention. Furthermore, the carbon-containing nutrients are preferably ones that are degraded and/or metabolised by the probiotic microorganism in the water-containing composition. The therapeutic efficacy of the probiotic microorganism in the probiotic composition is thought to depend on the metabolic activity of the microorganism, whereby the pH at the wound site is lowered to levels that inhibit proliferation of pathogenic organisms and inhibit proteolytic enzymes produced such pathogens.

The viability of the first probiotic microorganism (or additional probiotic microorganisms) is pH dependent, and hence the shelf life of the packaged probiotic composition is extended if the water-containing composition has a pH of at most pH 6.0. For example, the water-containing composition may have a pH of at most pH 5, preferably at most pH 4.5, and even more preferably at most pH 4.

In one embodiment the water-containing composition of the packaged probiotic composition has a pH in the range of pH 2-6. For example, the water-containing composition may have a pH in the range of pH 2.5 to 5, preferably in the range of pH 3.0 to 4.5, or even more preferably in the range of pH 3.0 to 4.0.

pH values mentioned in the context of the present invention pertain to the pH value of the relevant liquid at a temperature of 25 degrees C.

Additionally, the stability and/or viability of the probiotic microorganism is improved by keeping the probiotic microorganism in the same medium in which it has been fermented, and it is at least preferred that one or more of the fermentation product(s) is present in the water-containing composition.

Lactic acid is a fermentation product of lactic acid bacteria suitable for use in the packaged probiotic composition of the invention and may accordingly be present in the water-containing composition containing a first probiotic microorganism.

In some embodiments of the invention, the water-containing composition consists essentially of the fermentation broth obtained by fermenting the probiotic microorganism with carbon-containing nutrients until substantially all carbon-containing nutrients have been consumed. It is, however, possible to refine the fermentation broth. It may for example be preferred to remove components which are not physiologically acceptable, which interfer with the wound healing process or which inhibit the metabolism and/or growth of the probiotic microorganism once it has been applied to the wound or tissue to be treated.

Furthermore, lactic acid is also a suitable acid for regulating the pH of the water-containing composition.

In some embodiments the water-containing composition of the packaged probiotic composition comprises at least about 0.01% (w/w) lactic acid relative to the weight of the water-containing composition. For example, the water-containing composition may contain at least 0.05% (w/w) lactic acid, such as at least 0.1%

(w/w), preferably at least 0.2% (w/w), such as at least 0.4% (w/w), and even more preferred at least 0.6% (w/w), such as at least 1% (w/w).

The water-containing composition may for example contain an amount of lactic acid in the range of 0.01% - 2% (w/w), preferably in the range of 0.1% - 1.5% (w/w), and even more preferably in the range of 0.2% - 1% (w/w).

In the context of the present invention, the term "lactic acid" pertains to both the protonated and deprotonated state of lactic acid and therefore also encompassed lactate.

The water-containing composition, and the probiotic microorganisms contained therein, provides the rapid probiotic effect once the probiotic composition has been applied to the wound or tissue to be treated and thereby outcompete the pathogenic microorganisms of the wound or tissue.

The water-containing composition may for example be produced via a fermentation process, where probiotic microorganisms are mixed with water and nutrients and fermented at a suitable temperature, e.g. in the range of 20-37 degrees C, such as approx. 35 degrees C. Preferably, the fermentation continues until essentially all the carbon-containing nutrients have been consumed by the probiotic microorganisms.

The water-containing composition may furthermore contain other useful components such as salts, pH buffers and/or growth co-factors, which benefit the stability or the medical effect of the probiotic microorganism(s).

The probiotic composition contains a support agent which preferably has an open, and e.g. porous, structure that serves as a structural support for the water-containing composition comprising the first probiotic microorganism. Alternatively, the support agent may be a gel network or may have gel forming properties and form a hydrogel in combination with the water-containing composition.

The thickness of the support agent is sometimes a relevant feature when the support agent is in the form of fibres, a foam, or gel network which forms a hydrogel sheet. In some embodiments of the invention the support agent has a thickness, i.e. the distance between the side of the support agent which is to face the wound or tissue and its opposite side, is at most 5 cm, such as e.g. at most 4 cm, or such as e.g. at most 3 cm. For example, the support agent may have a thickness which is at most 2 cm.

In some embodiments of the invention the support agent has a thickness in the range of 0.1-5 cm, preferably in the range of 0.2-4 cm, and even more preferably in the range of 0.5-3 cm. For example, the support agent may have a thickness in the range of 1-2 cm.

The support agent may be even thinner, and in some embodiments of the invention the support agent has a thickness in the range of 0.05-2 cm, preferably in the range of 0.1-1.5 cm, and even more preferably in the range of 0.2-1 cm. For example, the support agent may have a thickness in the range of 0.3-0.8 cm.

Furthermore, the support agent preferably has water-absorbing and/or water-retaining properties. In some preferred embodiment of the invention the support agent is water-insoluble. In the context of the present invention a support agent is deemed water-insoluble if it has a solubility of at most 0.5 g/100 g water at 23 degrees C.

It may for example be preferred that the support agent comprises, or even consists of, one or more solid material(s). The support agent may for example comprise, or even consist of, one or more polymer materials.

The support agent preferably comprises, or even consists of, one or more biocompatible material(s), and is therefore preferably both suitable for supporting the viable probiotic microorganism in the composition and suitable for contacting wounds or tissue.

In some embodiments of the invention, the support agent does not bind irreversibly to the microorganism and does not prevent growth and mobility of the microorganism in the composition.

In other embodiments of the invention, the support agent binds at least some of probiotic microorganisms irreversibly.

The support agent may for example comprise, or even consist of, one or more polymer material(s) selected from the group consisting of a polyester, a polypropylene, a polyethylene, a polyacrylate, a polyvinylpyrrolidone, a polyvinylalcohol, a polyurethane, a polyvinylacetate, a polysaccharide, and a combination thereof.

Materials, which are not inherently hydrophilic, e.g. polyethylene or polypropylenes, may have a modified surface which renders them hydrophilic. Alternatively, materials, which are not inherently hydrophilic, may be mixed with materials that are hydrophilic.

It is generally preferred that the support agent retains its shape, structure and/or colour during storage prior to use, ensuring an adequate shelf-life of the packaged probiotic composition.

The inventor has discovered that for such embodiments, surprisingly, the stability of the support agent in the water-containing composition is important to obtain a long shelf-life of the probiotic composition. Furthermore, it may be desirable that the support agent does not degrade when applied to a wound. In such embodiments it is therefore preferred that the support agent is non-degradable by the water-containing composition.

For embodiments of the present invention where the water-containing composition contacts the support agent during storage, it is preferred that the support agent is non-degradable by the water-containing composition. For example, support agents which do not contain hydrolysable backbone bonds tend to be non-degradable.

Such support agents are preferably formed by one or more polymer material(s) which do not contain a polymer backbone comprising a bond-type that is prone to hydrolysis under the pH present in the water-containing composition. The water-containing composition typically has a mild acidic pH (pH 2-6).

Additionally, enzyme from the probiotic microorganism, which may be present in the water-containing composition, can contribute to the degradation of the support agent. It is therefore preferred that such support agents are formed by one or more polymer material(s) which do not contain a polymer backbone comprising a bond-type that is prone to hydrolysis in the presence of the specific enzymes of the water-containing composition.

In the context of the present invention, the term "non-degradable support agent" means that the support agent only displays very limited degradation or preferably no degradation at all during long term exposure to the water-containing composition.

The degradability of the support agent depends on the actual pH of the water-containing composition and the enzymes contained therein. It may therefore be necessary to perform an assay to test whether a specific support agent is non-degradable by the water-containing composition.

However, support agents which typically are non-degradable by water-containing compositions described herein may e.g. comprise, or even consist of, one or more polymer material(s) selected from the group consisting of an acid stable polyester, a polyurethane, a polypropylene, a polyethylene, a polyacrylate, a polyvinylpyrrolidone, a polyvinylalcohol, a polyvinylacetate, and a combination thereof.

If the support agent is a gel network and the probiotic composition comprises an amorphous hydrogel comprising both the support agent and the water-containing composition, it is preferred that the probiotic composition looses at most 25% of its viscosity when heated to a temperature of 35 degrees C for 3 days. The loss of viscosity is measured as:

$$\frac{\Pi_{before\ storage} - \Pi_{after\ storage}}{\Pi_{before\ storage}} * 100\%$$

where $\Pi_{before\ storage}$ is the dynamic viscosity of the amorphous hydrogel before the above-mentioned 3 days storage and $\Pi_{after\ storage}$ is the dynamic viscosity of the amorphous hydrogel after the storage. The dynamic viscosity is measured on a Brookfield viscometer at 23 degree Celsius.

If the support agent in the form of fibres, a foam or a gel network forming a hydrogel sheet, a support agent is deemed non-degradable if it has a weight loss of at most 10% w/w (dry weight) when submerged in the water-containing composition and heated to a temperature of 35 degrees C for 10 days.

The weight loss is measured as:

$$\frac{W_{before\ storage} - W_{after\ storage}}{W_{before\ storage}} * 100\%$$

where $W_{before\ storage}$ is the dry weight of the support agent before the above-mentioned 10 days storage and $W_{after\ storage}$ is the dry weight of the support agent after the storage. The dynamic viscosity is measured on a Brookfield viscometer at 23 degree Celsius.

The weight loss determination is based on a sample of the support agent which is has a dry weight of approx. 2 g and which has a maximum thickness of 2.0 cm. The dry support agent sample is weighed before storage ot obtain $W_{before\ storage}$, and is then submerged in a volume of water-containing composition corresponding to 20 times the outer volume of the support agent sample.

After storage at a temperature of 35 degrees C for 10 days, the support agent sample is separated from the water-containing composition, removing a much liquid as possible without damaging the support agent sample. Subsequently, the support agent sample is washed 3 times - each time with a volume of demineralised water corresponding to 20 times the outer volume of the support agent. The demineralised water should have a temperature of 23 degrees C. The support agent is contacted thoroughly with demineralised water and is allowed to stand for 20 minutes before as much water as possible is separated from the support agent. Once the demineralised water has been removed from the support agent, the next volume of clean demineralised water is brought in contact with the support agent sample as described above. When the support agent sample has been washed 3 times and most of the demineralised water has been removed, the moist support agent sample is placed in a heating cabinet providing a temperature of 70 degrees C and a relative humidity of 25% and is then dried for 24 hours. Finally, the weight of the dry support agent support is measured to obtain $W_{after\ storage}$.

The support agent may for example comprise, or even consist of, one or more material(s) in a form selected from the group consisting of fibres, a foam, a gel network, a gel-forming agent, and a combination thereof.

In some embodiments of the invention, the support agent is present in dry form in the packaged probiotic composition, meaning that the support agent is not in contact with any substantial amounts of water.

For example, the support agent is deemed dry if the combination of the support agent and any water absorbed by the support agent contains at most 10% (w/w) water relative to the total weight of the support agent and absorbed water.

Support agents in dry form may for example be prepared by freeze-drying or vacuum-drying a support agent, or a mixture of support agents, in wet form.

In some embodiments of the invention, the support agent is present in wet form in the packaged probiotic composition, meaning that the support agent is in contact with liquid water. In this case, the combination of the support agent and any water absorbed by the support agent contains more than 10% (w/w) water relative to the total weight of the support agent and absorbed water, and preferably at least 20% (w/w) water, and even more preferably at least 30% (w/w) water.

In some preferred embodiments of the invention, the support agent comprises, even consists of, fibres. Such fibres may for example be in the form of fabrics of woven fibres, non-woven fibres, or a combination thereof. The fibres may e.g. comprise, or even consist of, polymers such as polyester, polyurethane, polypropylene, polyethylene, polyacrylate, absorbent polyacrylate and a combination thereof.

The fibres may e.g. be attached to a polymer film, e.g. a polyester or polyurethane film.

A preferred type of fibres is super absorbent fibres (SAF) which may comprise, or even consist of, acrylic fibres. SAF fibres often have poor wet strength, why, when using SAF, it may be advantageous to blend the SAF with fibres that has a higher wet strength, such as e.g. polyester fibres.

In some embodiments of the invention, the fibres of the support agent comprise 25-95% (w/w) super absorbent fibres, preferably 35-85%(w/w), and even more preferably 50-80% (w/w) (w/w) super absorbent fibres relative to the total weight of fibres used in the support agent.

In some preferred embodiments of the invention the support agent comprises a mixture of superabsorbent fibres and low absorbent fibres.

In some preferred embodiments of the invention the support agent comprises fibres having a low or non-water absorbent core and a water absorbent shell. Such fibres may e.g. be Lanseal fibres from Toyobo, JP.

In the context of the present invention, the term "super absorbent fibre" means fibres that absorb at least 30 gram purified water per gram fibre.

In the context of the present invention, the term "low absorbent fibre" means fibres that absorb at most 10 gram purified water per gram fibre.

In some preferred embodiments of the invention, the support agent comprises, or even consists of, a foam.

A foam may e.g. comprise, or even consist of, polymers such as e.g. polyvinalcohol, polyacrylate, polyurethane, polyvinylacetate or a combination thereof.

In some preferred embodiments of the invention, the support agent comprises, or even consists of, a gel-forming agent.

In the context of the present invention, the term "gel-forming agent" pertains to agents which will form a hydrogel or a hydrogel-like viscous composition upon contact with the water-containing composition.

Useful hydrogel-forming agents typically include polyvinylpyrrolidone, polyvinylalcohol, polyacrylate, polyurethane, polyvinylacetate and the like.

In some preferred embodiments of the invention, the support agent comprises, or even consists of, a gel network.

In the context of the present invention, the term "gel network" pertains to the network of cross-linked gel-forming agents, which form the skeleton of a hydrogel.

In some preferred embodiments of the invention, the probiotic composition comprises, or even consists of, a hydrogel comprising the support agent and water. In this case the support agent comprises, or even consists of, the gel network.

A hydrogel may take the form of a hydrogel sheeti.i.e. a hydrogel containing a continuous matrix of cross linked polymers, thereby forming a sheet-like hydrogel-structure. In this case the gel network typically is continuous network of cross-linked gel-forming agents.

Alternatively, the hydrogel may take the form of an amorphous hydrogel. In this case the gel network typically contains numerous network fragments of cross-linked gel-forming agents. When hydrated, the network fragments can move relative to each other and there provide the amorphous hydrogel with liquid-like properties.

In yet an alternative, the hydrogel may be a highly viscous liquid having properties similar to an amorphous hydrogel.

Where the probiotic composition as such is an amorphous hydrogel comprising the first probiotic microorganism, or where the support agent forms an amorphous hydrogel with the water-containing composition, the amorphous hydrogel may have a dynamic viscosity of at least 50000 cP. For example, the amorphous hydrogel may have a dynamic viscosity of at least $1*10^5$ cP, preferably at least $3*10^5$ cP, such as at least $6*10^5$ cP, and even more preferably at least $9*10^5$ cP, such as at least $1*10^6$ cP. The dynamic viscosity is measured on a Brookfield viscometer at 23 degree Celsius.

Alternatively, the amorphous hydrogel may have a dynamic viscosity in the range of $5*10^4$ - $2*10^6$ cP, preferably in the range of $1*10^5$ - $1.5*10^6$ cP, and even more preferably in the range of $5*10^5$ - $1*10^6$ cP.

Where the packaged probiotic composition comprises a hydrogel comprising the support agent and the water-containing composition, the hydrogel preferably contains at least 50% (w/w) water. Alternatively, the hydrogel may contain at least 60% (w/w), such as at least 70% (w/w), preferably at least 80% (w/w), such as at least 90% (w/w), or even more preferably at least 95% (w/w), such as at least 97% (w/w) water. Alternatively, the hydrogel may contain between about 50% - 60% (w/w) water, or 60% - 70% (w/w) water, or 70% - 80% (w/w) water, or 80% - 90% (w/w) water, 90% - 97% (w/w) water or 95% - 99% (w/w) water.

In some preferred embodiments of the invention, the probiotic composition is a wound dressing.

In some embodiments of the invention, where the probiotic composition is a wound dressing, the support agent comprises, or even consists of, one or more material(s) in a form selected from the group consisting of fibres, a foam, a gel network, or gel-forming agent, and a combination thereof.

The probiotic composition may for example be a so-called pad dressing, which comprises, or even consists of, the support agent, the water-containing compositions, and optionally also one or more additional layers such as moisture barrier layer. Pad dressings typically need a bandage or other means to keep them in place on the treatment site.

Preferred support agents for a pad dressing may contain one or more material(s) in the form of fibres, a foam, or a gel network. The support agent for the pad may furthermore contain gel forming agents - particularly when the support agent is used in dry form.

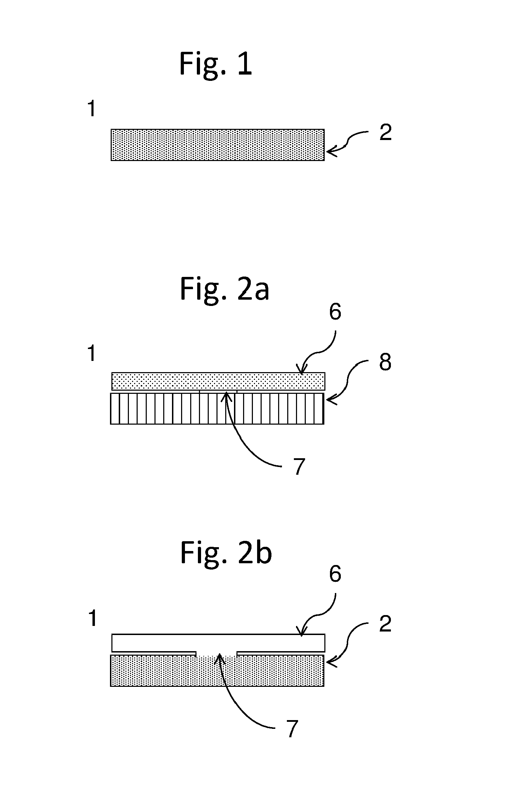

An exemplary embodiment of a probiotic composition (1) which is a pad wound dressing is illustrated in Figure 1, which contains a schematic illustration of a cross section of a support agent containing the water-containing composition (2).

In some preferred embodiments of the invention, the water-containing composition contacts the support agent. This is for example the case when the probiotic composition is a hydrogel or when the probiotic composition comprises, or even consists of, a wound dressing soaked with the water-containing composition.

However, in other preferred embodiments of the invention, the water-containing composition does not contact the support agent while the probiotic composition is in a first state, but where the probiotic composition can be transformed to a second state where the water-containing composition contacts the support agent.

The first state is typically the state where the probiotic composition during storage is in the unopened container. The transformation to the second state preferably occurs during or after the opening of the container, or immediately before opening the container.

In some embodiments of the invention, the probiotic composition comprises a sealed reservoir containing the water-containing composition and a conduit which will allow the water-containing composition to contact the support agent when the sealed reservoir is opened. The sealed reservoir is typically opened during the above-mentioned transformation.

An exemplary embodiment of this is schematically illustrated in Figures 2a and 2b. In addition to the components described in Figure 1, this probiotic composition furthermore contains a reservoir (6) in which the water-containing composition is sealed during storage. The reservoir (6) is preferably connected to the support agent (8) via a conduit (7). In Figure 2a, the probiotic composition is in its first state, i.e. the water-containing composition is kept in the reservoir (6), separate from the support agent (8). The probiotic composition of this embodiment can be transformed into a second state, schematically illustrated in Figure 2b, where the water-containing composition of the reservoir (6) has been transferred to the support agent (2) which now contains both water and first probiotic microorganisms from the water-containing composition.

The transformation typically involves opening the reservoir (6) and allowing the water-containing composition to flow via the conduit (7) into the support agent (8).

The reservoir may e.g. be opened by pressing part of the reservoir against a protrusion which perforates the reservoir wall and allows the water-containing composition to flow into the support agent. Alternatively, the reservoir may be designed to break in a controllable fashion when it is bent, and the breakage allows the water-containing composition to flow to and be absorbed by the support agent.

The packaged probiotic composition, when used as a wound dressing, may furthermore comprise an adhesive layer that serves to form a contact layer between the skin surrounding the wound, or the wound as such, and the applied dressing. The adhesive layer is preferably permeable or semi-permeable allowing exudate to evaporate out of the dressing. Preferred adhesive layers comprise absorbent adhesives (such as Comfeel transparent dressing from Coloplast A/S, Denmark), silicone adhesives, polyacrylate adhesives or polyurethane adhesives.

The probiotic composition may furthermore comprise an adhesive layer. Such an adhesive layer should preferably be capable of adhering the probiotic composition to the site where it is to be active.

A top layer may form the outer surface of the probiotic composition most remote from the wound contact surface that serves to prevent leakage of wound exudate from the dressing and to prevent entry of contaminants into the wound dressing. Preferably the top layer is able to breathe, being permeable to both oxygen and to moist vapour. A vapour transmission rate of at least 1000 grams per day per square meter per 24 hours is preferred. Suitable materials for the top layer are polyurethane films.

In one embodiment, the water-containing composition is not in contact with the support agent prior to use (i.e in its unopened state prior to use), and where the step of opening the packaging causes a transformation of the probiotic composition when the water-containing composition contacts the support agent.

The support agent may for example be attached to the top layer, either directly or indirectly, through one or more other materials.

The probiotic composition may furthermore comprise a bottom layer. A bottom layer is preferably highly water permeable and preferably porous. In some embodiments of the invention the bottom layer has pores or openings which are sufficiently large to allow the water-containing composition including the first probiotic microorganism to flow though the bottom layer into the wound or tissue to be treated. However, it may be preferred that the pores or openings are sufficiently small to retain the support agent. This embodiment is particularly preferred when the support agent must not contact the wound or tissue to be treated.

In some preferred embodiments of the invention, the probiotic composition comprises a top layer, a bottom layer and an adhesive, and wherein said support agent and said water-containing composition are located in a cavity which is at least partly defined by the top layer and/or the bottom layer, and wherein the adhesive is arranged such that the probiotic composition can adhere to a skin surface in such a way that the bottom layer contacts the wound surface.

An exemplary embodiment of this is shown in Figures 1 and 2. Figure 1 shows the components used to produce the probiotic composition: A top layer (3), a support agent which contains, and contacts, the water-containing composition (2), a bottom layer (4) and an adhesive layer (5). The probiotic composition may be produced by placing the support agent (2), which contains the water-containing composition, in a cavity formed by the top layer (3) and closing the cavity by attaching the bottom layer (4) to at least a part of the top layer (3), which part surrounds the cavity. The attachment may be performed by use of traditional attachment techniques, such as welding or use of an adhesive.

Additionally, if the top layer does not have sufficient adhesive characteristics itself, an adhesive layer (5) may be present on at least some of the parts of surface the top layer which is to contact the skin surrounding the wound or tissue to be treated. The adhesive layer (5) may furthermore be attached to a non-sticking sheet (not shown in the figures) which prevents the adhesive layer (5) from sticking to the packaging of the packaged probiotic composition, and which has to be removed from the probiotic composition prior to use.

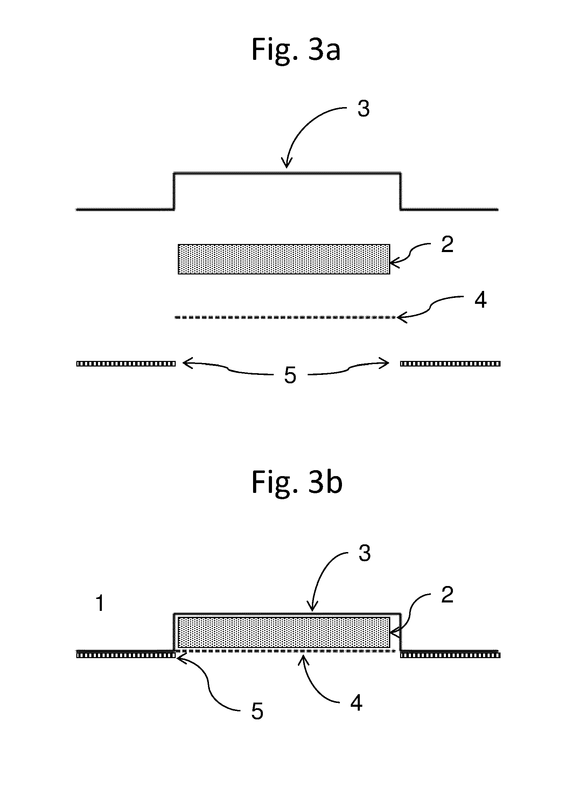

An exemplary embodiment of this is schematically illustrated in Figures 3a and 3b. In addition to the components described in Figures 1 and 2, this probiotic composition furthermore contains a reservoir (6) in which the water-containing composition is sealed during storage. The reservoir (6) is preferably connected to the support agent (8) via a conduit (7). In Figure 3a, the probiotic composition is in its first state, i.e. the water-containing composition is kept in the reservoir (6), separate from the support agent (8). The probiotic composition of this embodiment can be transformed into a second state, schematically illustrated in Figure 3b, where the water-containing composition of the reservoir (6) has been transferred to the support agent (2) which now contains both water and first probiotic microorganisms from the water-containing composition.

The transformation typically involves opening the reservoir (6) and allowing the water-containing composition to flow via the conduit (7) into the support agent (8).

As stated above, the reservoir may e.g. be opened by pressing part of the reservoir against a protrusion which perforates the reservoir wall and allows the water-containing composition to flow into the support agent. Alternatively, the reservoir may be designed to break in a controllable fashion when it is bent, and the breakage allows the water-containing composition to flow to and be absorbed by the support agent.

The probiotic composition preferably contains sufficient water to be able of donate water to the wound or tissue to be treated.

In one embodiment, the probiotic composition contains at least 0.1 mL of the water-containing composition. For example, the probiotic composition may contain at least 0.5 mL of the water-containing composition, such as at least 1 mL, or even at least 3 mL of the water-containing composition.

Even higher amounts of amounts of water-containing composition may be used. Thus in some embodiments of the invention, the probiotic composition contains at least 50 mL of the water-containing composition. For example, the probiotic composition may contain at least 100 mL of the water-containing composition, such as at least 200 mL, or even at least 300 mL of the water-containing composition.

In some preferred embodiments of the invention, the probiotic composition contains in the range of 0.1-100 mL of the water-containing composition, preferably in the range of 0.2-50 mL, preferably in the range of 0.5-30 mL, and even more preferably in the range of 1-20 mL of the water-containing composition.

Even higher amounts of amounts of water-containing composition may be used. Thus in some embodiments of the invention, the probiotic composition contains an amount of the water-containing composition in the range of 50-500 mL. For example, the probiotic composition may contain an amount of the water-containing composition in the range of 100-450 mL, preferably in the range of 150-400 mL, or even in the range of 200-350 mL.

An advantage of the probiotic composition of the present invention is that it is particularly suited for dry wounds as it both donates water to the wound and provides a controlled microbial environment containing probiotic microorganisms.

In some preferred embodiments, the support agent of the packaged probiotic composition may have absorbed an amount of water, e.g. provided by the water-containing composition, corresponding to at least 50% of the water holding capacity of the support agent. For example, the support agent may contain an amount of water corresponding to at least 75% of its water holding capacity, preferably at least 90%, and even more preferred at least 95% its water holding capacity. In embodiments where the support agent is kept separate from the water-containing composition during storage, the above water contents describe situations where the support agent has been contacted by the water-containing composition prior to the application of the probiotic composition to the wound or tissue.

The water holding capacity is measured according to EN 13726-1 section 3.2 where a given weight of wound dressing is submerged into saline solution followed by a weight measurement.

In some preferred embodiments of the invention the weight ratio between the support agent and the water-containing composition is at most 1:1, preferably at most 1:2, and even more preferred at most 1:5. For example, the weight ratio between the support agent and the water containing composition may be at most 1:10, preferably at most 1:20, and even more preferred at most 1:40, such as approx. 1:50.

In some preferred embodiments of the invention the weight ratio between the support agent and the water-containing composition is in the range of 1:1-1:50, preferably in the range of 1:2-1:30, and even more preferably in the range of 1:4-1:20.

The probiotic composition has been packaged in a suitable container and is therefore a packaged probiotic composition. The container is important for maintaining a controlled microbial environment in the probiotic composition and for keeping the water in the water-containing composition.

The container in which the probiotic composition is packaged, typically includes a material which provides a protective sterile barrier layer having a water permeability of at most 10 g/m$^2$/24 hours (measured according to EN 13726-2, section 3.2). Suitable materials are typically selected from plastics, aluminium foil, plastic laminates, optionally bonded with an adhesive (e.g. polyurethane). Suitable plastics include: PET, PE, LLDPE, CPP, PA, PETP, METPET and Tyvek.

Several container types may be useful for packaging the probiotic composition. Non-limiting examples of useful containers are welded films such as aluminum-films, boxes, bags, trays, cans, and wrappers.

An advantage of the present invention is that the packaged probiotic composition has a very long shelf- life. In some embodiments the packaged probiotic composition has a shelf-life of at least 6 month when kept at a temperature of 23 degrees C and a relative humidity of 50%. For example, the packaged probiotic composition may have a shelf-life of at least 9 month, such as at least 12 months, and even more preferably at least 18 months when kept at a temperature of 23 degrees C and a relative humidity of 50%.

The shelf life at a given temperature and humidity is determined as the time running from production of the packaged probiotic composition to the first occurrence of at least one of the following events during storage:

- Gas bubbles can be observed by visual inspection of the probiotic composition
- The probiotic composition has lost more than 99% of the viable probiotic microorganisms relative to the number of colony forming units (cfu) of probiotic microorganisms present in the newly produced probiotic composition,
- Degradation of the support agent can be observed by visual inspection, and
- Discolouration of the support agent can be observed by visual inspection.

The invention provides a method of producing a packaged probiotic composition, comprising the steps of:

a) providing a water-containing composition containing a viable first probiotic microorganism,
b) providing a support agent as defined herein,
c) optionally, contacting the support agent with the water-containing composition,
d) packaging the combination of the support agent and the water-containing composition in a suitable container.

The packaged probiotic composition is preferably manufactured under aseptic conditions, and may for example employ a class 100,000 clean room.

Furthermore, the packaging of step d) is performed under conditions that minimize loss of viability of the probiotic microorganism(s). Thermal processes such as welding should be applied carefully to avoid damaging the probiotic microorganisms.

Yet an aspect of the present invention pertains to the packaged probiotic composition of the invention is for prophylactic or therapeutic use for an animal or human. In particular, the probiotic composition is for use as a wound dressing for treatment of wounds or tissues and for treatment and prevention of colonization or infection by pathogenic microorganisms at the wound or tissue site.

A further aspect of the present invention pertains to a method of treating an animal or human subject having a colonized wound or tissue, or being at risk of having a colonized wound or tissue, the method comprising the steps of:

1) providing a packaged probiotic composition;

2) opening the container in which the probiotic composition has been packaged, and

3) applying the probiotic composition to the colonized wound or tissue.

The probiotic composition should preferably be applied so that the first probiotic microorganism is at least in liquid communication with the wound or tissue to be treated. It may furthermore be preferred that the first probiotic microorganism contacts the wound or tissue to be treated.

The present invention has been described above with reference to specific embodiments. However, other embodiments than the above described are equally possible within the scope of the invention. The different features and steps of various embodiments and aspects of the invention may be combined in other ways than those described herein unless it is stated otherwise.

## EXAMPLES

### Example 1 a Preparation of a water-containing composition

A water-containing composition was prepared by mixing 800 grams of sugar from Danisco, 16000 grams of 35 degrees C hot water and a probiotic blend of 1 gram Lactobacillus Acidophilus, 1 gram of Bifidobacterium Animalis and 1 gram of Lactubacillus Casai from Chr. Hansen.

The composition was allowed to ferment for 3 weeks until all sugar had been metabolised by the probiotic blend. The pH of the resulting water-containing composition was pH 4.

### Example 1b A water-containing composition

1 litre of "Vita Biosa Probiotic urter" having a pH of 3.4 was purchased from Biosa Danmark and used as an alternative water-containing composition.

### Example 2 Preparation of packaged wound dressings

A PVA foam from Mondomed (8x12x0.6 cm) was impregnated with the water-containing composition from example 1b by dipping the foam into the water-containing composition. After 1 minute, the foam was removed and excess liquid was allowed to drip off. The sample was called 2a.

A non-woven fabric comprising super absorbent fibres (SAF) from Technical Absorbent (comprising 75% SAF of polyacrylate and 25% polyester fibres) was impregnated with the water-containing composition by dipping the non-woven into the water-containing composition 1b. After 1 minute, the foam was removed and excess liquid was allowed to drip of. The sample was called 2b.

An Alginate Wound dressing from Kanglidi Medical (a non-woven fabric) was impregnated with the water-containing composition by dipping the non-woven into the water-containing composition 1b. After 1 minute, the non-woven was removed and excess liquid was allowed to drip of. The sample was called 2c.

A Chitosan Wound dressing from Kanglidi Medical (a non-woven fabric) was impregnated with the water-containing composition by dipping the non-woven into the water-containing composition 1b. After 1 minute, the non-woven was removed and excess liquid was allowed to drip of. The sample was called 2d.

All samples were subsequently packed and sealed in zipper pouches and subjected to the testing described in Example 4.

### Example 3 Preparation of packaged amorphous hydrogels

20 grams of superabsorbent polyacrylate particles from BASF was blended with 500 grams of probiotic composition and 500 grams of saline water (0.9% NaCl). The amorphous hydrogel was packed in a syringe and called 3a.

35 grams of Chitosan Primex (Chitoclear high molecular weight) was blended with 500 grams of probiotic composition and 500 grams of saline water (0.9% NaCl). The amorphous hydrogel was packed in a syringe and called 3b.

The two samples were subsequently packed in syringes and subjected to the testing described in Example 5.

### Example 4 Comparative testing - wound dressings

The wound dressings from example 2 were tested in relation to stability and how the water containing composition influenced the stability and appearance of the support agent. The samples were stored at 35 degree Celcius for 48 hours. After the 48 hours, the samples were visually inspected in relation to coloration, formation of gas in the pouch, visual evaluation of degradation of the support agent and formation of gas bobbles in the wound dressing. The results are summarized in Table 1.

Table 1 Comparison of wound dressings

| Sample | Visual inspection | | | |
| --- | --- | --- | --- | --- |
| | Color | Gas formation | Other comments | Conclusion |
| 2a | Off White to light brown | No | Nice even appearance, same color as before heating. | The support agent is not affected by the water-containing composition, and is concluded to be useful as support agent. |
| 2b | Light brown | No | Nice even appearance, same color as before heating. | The support agent is not affected by the water-containing composition, and is concluded to be useful as support agent. |
| 2c | Brown | No | Slightly more dark color after testing, but with an even color distribution on the sample. Has loss some liquid. | The support agent is affected by the water-containing composition to such a degree that it is not useful as a support agent. |
| 2d | Dark brown | Yes, but minimal | Partly soluble, partly gelled (soft gel). Uneven color distribution on the sample. | The support agent is affected by the water-containing composition to such a degree that it is not useful as a support agent. |

Based on the above test, it is concluded that samples 2a and 2b are stable and useful as a support agent. Samples 2c and 2d, however, are significantly affected by the water-containing composition and are therefore unsuitable as support agents in probiotic compositions where the water-containing composition contacts the support agent during storage of the probiotic composition.

**Example 5 Comparative testing - amorphous hydrogels**

The samples were stored at 35 degree Celcius for 48 hours. After the 48 hours, the samples were visually inspected in relation to coloration, formation of gas in the syringes, visual evaluation of degradation of the support agent and formation of gas bubbles in the hydrogel. The results are summarized in Table 2.

Table 2 Comparison of hydrogels

| Sample | Visual inspection | | | |
| --- | --- | --- | --- | --- |
| | Color | Gas formation | Other comments | Conclusion |
| 3a | Light brown | No | The sample has a nice even appearance, same color as before heating. Seems to maintain its original viscosity. | The support agent is not affected by the water containing composition, and is concluded to be useful as support agent. |
| 3b | Dark brown | No | More dark color after testing, the sample seems to have lost some viscosity. | The support agent is affected by the water containing composition to such a degree, that the sample is not useful as support agent. |

Sample 3a is evaluated to be useful as a support agent in the present invention, while sample 3b is found not useful as it is affected significantly by the water-containing composition and shows clear signs of degradation. 3b is therefore unsuitable as support agent in probiotic compositions where the water-containing composition contacts the support agent during storage of the probiotic composition.

**Claims**

1.  A packaged probiotic composition, the probiotic composition comprising:

    - a water-containing composition containing a viable first probiotic microorganism, and
    - a support agent.

2.  The packaged probiotic composition according to claim 1, wherein the water-containing composition has a pH in the range of pH 2-6.

3.  The packaged probiotic composition according to claim 1 or 2, wherein the support agent is water-insoluble.

4.  The packaged probiotic composition according to claim 1 or 2, wherein the first probiotic microorganism is a bacterium or a fungus.

5.  The packaged probiotic composition according to any of the preceding claims, wherein the probiotic composition comprises at least 0.1 mL water-containing composition.

6.  The packaged probiotic composition according to any of the preceding claims, wherein the weight ratio between the support agent and the water-containing composition is at most 1:1.

7.  The packaged probiotic composition according to any of the preceding claims, wherein the total amount of carbon-containing nutrients having a molecular weight of at most 5000 g/mol in the water-containing composition is at most 0.5% (w/w) relative to the weight of the water-containing composition.

8.  The probiotic composition according to any the preceding claims, wherein the support agent comprises one or more material(s) in a form selected from the group consisting fibres, a foam, a gel network, a gel-forming agent, and a combination thereof.

9.  The packaged probiotic composition according to any of the preceding claims, wherein the support agent is a water-insoluble gel forming agent.

10. The packaged probiotic composition according to any of the preceding claims, wherein the water-containing composition contacts the support agent.

11. The packaged probiotic composition according to any of the claims 1-35, wherein the water-containing composition does not contact the support agent while the probiotic composition is in a first state, but wherein the probiotic composition can be transformed into a second state where the water-containing composition contacts the support agent.

12. The packaged probiotic composition according to any of the preceding claims, the packaged probiotic composition having a shelf-life of at least 3 months when kept at a temperature of 23 degrees C and a relative humidity of 50%.

13. A method of producing a packaged probiotic composition, the method comprising the steps of:

    a) providing a water-containing composition containing a viable first probiotic microorganism,
    b) providing a support agent,
    c) optionally, contacting the support agent with the water-containing composition, and
    d) packaging the combination of the support agent and the water-containing composition in a suitable container.

14. A method of treating a human or animal subject having a colonized wound or tissue, or being at risk of having a colonized wound or tissue, the method comprising the steps of:

1) providing a packaged probiotic composition according to any of the claims 1-12,
2) opening the container in which the probiotic composition has been packaged, and
3) applying the probiotic composition to the colonized wound or tissue.

15. The probiotic composition according to any of the claims 1-12 for use in treatment or prevention of colonized wound or tissue.

# Fig. 1

1

2

# Fig. 2a

6

1

8

7

# Fig. 2b

6

1

2

7

# Fig. 3a

# Fig. 3b

## Fig. 4a

## Fig. 4b

**EP 2 596 776 A1**

Europäisches Patentamt
European Patent Office
Office européen des brevets

# EUROPEAN SEARCH REPORT

Application Number

EP 11 19 0803

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/000123 A1 (UNIV DE CONCEPCION [CL]; CASTRO INOSTROZA ERICA [CL]; BORQUEZ YANEZ RO) 6 January 2011 (2011-01-06) * page 4, line 15 - page 5, line 37 * ----- | 1-15 | INV. A61F13/00 A61L15/36 |
| X | WO 00/71139 A2 (REID GREGOR [CA]; BRUCE ANDREW W [CA]) 30 November 2000 (2000-11-30) * page 7, line 7 - page 8, line 2 * * page 18, line 15 - line 20 * ----- | 1-15 | |

| TECHNICAL FIELDS SEARCHED (IPC) |
|---|
| A61F A61L |

The present search report has been drawn up for all claims

| Place of search | Date of completion of the search | Examiner |
|---|---|---|
| The Hague | 15 June 2012 | Westberg, Erika |

EPO FORM 1503 03.82 (P04C01)

## ANNEX TO THE EUROPEAN SEARCH REPORT
## ON EUROPEAN PATENT APPLICATION NO.

EP 11 19 0803

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2012

| Patent document cited in search report | | Publication date | Patent family member(s) | | Publication date |
|---|---|---|---|---|---|
| WO 2011000123 | A1 | 06-01-2011 | EP 2450062 A1<br>WO 2011000123 A1 | | 09-05-2012<br>06-01-2011 |
| WO 0071139 | A2 | 30-11-2000 | AU 4905400 A<br>CA 2374938 A1<br>WO 0071139 A2 | | 12-12-2000<br>30-11-2000<br>30-11-2000 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008074331 A
- WO 0061201 A